# EUROPEAN PATENT SPECIFICATION

(11) **EP 0 410 813 B1**
(45) Date of publication and mention of the grant of the patent: **17.01.1996**
(21) Application number: 90308319.4
(22) Date of filing: 30.07.1990
(51) Int. Cl.: C12P 21/08

(54) **Anti-human BCDF monoclonal antibody and immunoassay using the same**
Monoklonaler Antikörper gegen menschliches BCDF und Immuntestverfahren unter dessen Verwendung
Anticorps monoclonal contre le BCDF humain et immuno-analyse l'utilisant

(30) Priority: 28.07.1989 JP 194334/89
(43) Date of publication of application: 30.01.1991
(73) Proprietor: FUJIREBIO INC., Tokyo 161 (JP); Kishimoto, Tadamitsu, Tondobayashi-shi Osaka-fu (JP)
(72) Inventor: Shimamura,Toshiro, c/o Ajinomoto Co.Inc., Kawasaki-shi, Kanagawa 210 (JP); Takahara, Yoshiyuki, c/o Ajinomoto Co.Inc., Kawasaki-shi, Kanagawa 210 (JP); Honda, Hideo, Higashimurayama-shi, Tokyo 189 (JP); Yokota, Masataka, Hachioji-shi, Tokyo 193 (JP); Kishimoto, Tadamitsu, Tondabayashi-shi, Osaka 584 (JP)
(74) Representative: Paget, Hugh Charles Edward

(56) References cited:
- EP-A- 0 220 063
- EP-A- 0 399 429
- HYBRIDOMA, vol. 8, no. 5, March 1989, New York, NY (US); D. NOVICK et al., pp. 561-567/
- DERWENT ABSTRACTS, 12 May 1987, Derwent Publications Ltd., London (GB); AN 87- 168184/
- CLIN. EXP. IMMUNOLOGY, vol. 75, no. 1, January 1989, Oxford (GB); N. JACKSON et al., pp. 93-99/
- BIOCHEMICAL & BIOPHYSICAL RESEARCH COMMUNICATIONS, vol. 165, no. 2, 15 December 1989, New York, NY (US); N. IDA et al., pp. 728-734/

## Description

This invention relates to an anti-human BCDF monoclonal antibody and immunoassay for quantifying human BCDF in a sample.

Human BCDF also known as human B cell stimulating factor 2 (BSF-2), or interleukin-6 (IL-6), is a biological substance which is closely associated with the immunomodulating system produced by the immunological cells and its entire amino acid sequence has been determined (Nature, vol. 324, p 73, 1980).

It is known that human BCDF exhibits various biological activities. For example, human BCDF induces the production of antibodies by the activated B cells (Journal of Experimental Medicine, vol. 167, p.332, 1988), and it also induces the differentiation of T cells (Journal of Immunology, vol. 140, p.508, 1988). It is also known that human BCDF induces the synthesis of the acute phase protein emerging in the initial phase of inflammation of liver cells (FEBS Letters, vol. 221, p.18, 1987) and it acts on the blood stem cells to induce the formation of colonies of pluripotent hematopoietic stem cells (Proceedings of National Academy of Science, vol. 84, p.9035, 1987). On the other hand, patients suffering from atrial myxoma present Systemic autoimmune syndromes. It is known that the primary tumor cells in the atrial myxoma produce human BCDF and the autoimmune syndrome of atrial myxoma is improved by removing the primary tumor cells (Proceedings of National Academy of Science, vol. 82, p.5490, 1985). It is also known that the BCDF level of joint fluid in rheumatoid arthritis patients is significantly higher than in normal humans (Medical Immunology, vol. 15, p. 195, 1988), and human BCDF is abnormally produced in the hypertrophic lymph node in Castleman's syndrome (Medical Immunology, vol. 15, p. 197, 1988). From these observations, it is supposed that human BCDF closely relates to some of the autoimmune diseases. It is also known that in the rejection reaction against a transplanted organ, human BCDF level is temporarily increased immediately before the rejection reaction, that human BCDF level is high in the lesion under inflammation, that tumor cells collected from the patients suffering from a multiple myeloma produce human BCDF and that the growth of the tumor cells is observed by adding human BCDF thereto (Nature, vol. 332, p.83, 1988).

Thus, human BCDF plays important roles in the immunomodulation in the body, and abnormally high production of human BCDF is closely associated with the above-described autoimmune diseases, inflammation and some cancers.

Thus, it is believed that determination of human BCDF level in the blood or other body fluid is important for diagnosis and monitoring of patients suffering from these diseases. Further, it is expected that reduction of the activities of human BCDF which is produced in the abnormally high amounts will be an efficient treatment of these diseases.

Conventional methods for determining the human BCDF level include methods in which the cells of which growth or differentiation depends on the existence of the human BCDF are employed, and immunoassays employing anti-human BCDF antibodies (European Journal of Immunology, vol. 18, p. 951, 1988).

With the former conventional methods, the detection sensitivity is as high as several hundred fg/ml. However, since they are bioassays, the test results for determining the human BCDF level in the blood or other biological fluids are affected by various substances in the blood or the biological fluids, so that the methods are difficult to reproduce.

On the other hand, with the conventional immunoassays, the detection sensitivity is too low to detect BCDF in the blood. In view of the fact that the human BCDF level in the blood of normal humans is expected to be about 10 pg/ml, the sensitivity of conventional immunoassays for quantifying human BCDF is unsatisfactory. Further, in conventional immunoassays, polyclonal antibodies are employed as the secondary antibody. In general, the polyclonal antibody is a mixture of a number of antibodies, so that it is impossible to reproduce the same polyclonal antibody with the same properties. Further, a polyclonal antibody cannot be produced in a large amount in one batch and so it is impossible to produce the polyclonal antibody on an industrial scale.

If an immunoassay system exists in which not only the first antibody but also the second antibody is a monoclonal antibody, the human BCDF level can be determined with high sensitivity in samples where the blood contains various substances. Further, since monoclonal antibodies can be produced by culturing hybridomas, and the same monoclonal antibody can be produce repeatedly, the immunoassay system can be produced on an industrial scale while still assuring the identity of the system. To provide such an immunoassay system, at least two anti-human BCDF monoclonal antibodies in which the corresponding epitopes are different necessary.

EP 312 996 discloses monoclonal antibodies to human BCDF that are specific for one epitope of BCDF.

EP 399 429, cited under EPC Art 54 (3), discloses a monoclonal antibody specific for human BCDF that is capable of neutralizing 50% of BCDF activity when present in a 10-fold excess.

Accordingly, one object of the present invention is to provide an anti-human BCDF antibody with high specificity in which the epitope is different from that recognized by a conventional anti-human BCDF monoclonal antibody.

Another object of the present invention is to provide an immunoassay utilizing the anti-human BCDF monoclonal antibody of the present invention.

Accordingly, the present invention provides an anti-human BCDF monoclonal antibody having the epitope specificity of an antibody obtained from hybridoma HH61-8 deposited as FERM BP-3029 or from hybridoma HH61-10 deposited as FERM BP-3030.

That is, the present invention provides an anti-human BCDF monoclonal antibody as obtainable from the hybridoma HH61-8 deposited as FERM BP-3029 or from the hybridoma HH61-10 deposited as FERM BP-3030. This antibody is capable of specifically binding to human BCDF, recognizes an epitope which is different from that recognized by antihuman BCDF monoclonal antibodies MH166 and αBSF2-77, and can neutralize human BCDF activity.

The present invention also provides an immunoassay for determining human BCDF level in a sample comprising reacting human BCDF with the anti-human BCDF monoclonal antibody of the present invention and determining the amount of human BCDF specifically bound to said anti-human BCDF monoclonal antibody.

Accordingly, the present invention provides a anti-human monoclonal antibody, having high specificity which recognizes an epitope that is different from that recognized by the conventional anti-human BCDF monoclonal antibodies. Since the anti-human BCDF monoclonal antibody of the present invention has high specificity to human BCDF, and since it recognizes an epitope that is different from that recognized by the conventional anti-human monoclonal antibodies, an immunoassay system was provided for quantifying human BCDF in a sample which utilizes two anti-human BCDF monoclonal antibodies. Thus, by the present invention, a highly sensitive immunoassay system was provided for quantifying human BCDF, which is reproducible on an industrial scale. The present invention greatly contributes to the diagnosis and monitoring of the diseases associated with human BCDF, as well as to the treatment of such diseases.

In the accompanying drawings:
Fig. 1 graphically shows the activities of the monoclonal antibodies of the present invention for neutralizing the human BCDF activities; and
Fig. 2 graphically shows the results of the enzyme immunoassay utilizing the anti-human BCDF monoclonal antibody of the present invention.

As mentioned above, the anti-human monoclonal antibody of the present invention has the following properties:
(i) it specifically binds to human BCDF;
(ii) it recognizes an epitope which is different from that recognized by anti-human BCDF monoclonal antibodies MH166 and αBSF2-77; and
(iii) it neutralizes human BCDF activity.

Examples of the anti-human BCDF monoclonal antibody of the present invention include HH61-8 and HH61-10 which were actually obtained in the working examples described below and which were deposited with Fermentation Research Institute of Japan in accordance with Budapest Treaty under the accession numbers FERM BP-3029 and FERM BP 3030, respectively. The monoclonal antibodies HH61-8 and HH61-10 have the following properties:
I) Monoclonal Antibody HH61-8
   i) Immunoglobulin Class: IgG
   ii) Molecular Weight (determined by SDS-PAGE): 150,000 daltons
   iii) Molecular Absorption Coefficient E_{280 nm} (determined by the measurement of absorbance at 280 nm and of the dry weight): 14.0
   iv) It specifically binds to human BCDF.
   v) It recognizes an epitope which is different from that recognized by anti-human BCDF monoclonal antibodies MH 166 (deposited with Fermentation Research Institute of Japan in accordance with the Budapest Treaty under the accession number FERM BP-1972) and αBSF2-77 (deposited with Fermentation Research Institute of Japan in accordance with the Budapest Treaty under the accession number FERM BP-1975).
   vi) It neutralizes human BCDF activities.
   vii) It inhibits the binding of human BCDF to BCDF receptors.
II) Monoclonal Antibody HH61-10
   i) Immunoglobulin Class: IgG
   ii) Molecular Weight (determined by SDS-PAGE): 150,000 daltons
   iii) Molecular Absorption Coefficient E_{280 nm} (determined by the measurement of absorbance at 280 nm and of the dry weight): 14.0
   iv) It specifically binds to human BCDF.
   v) It recognizes an epitope which is different from that recognized by anti-human BCDF monoclonal antibodies MH 166 (deposited with Fermentation Research Institute of Japan in accordance with the Budapest Treaty under the accession number FERM BP-1972) and αBSF2-77 (deposited with Fermentation Research Institute of Japan in accordance with the Budapest Treaty under the accession number FERM BP-1975).
   vi) It neutralizes human BCDF activities.
   vii) It inhibits the binding of human BCDF to BCDF receptor.

Since the epitope recognized by the anti-human monoclonal antibody of the present invention is different from that recognized by conventional anti-human BCDF monoclonal antibodies, it is apparent that the monoclonal antibody of the present invention is different from the conventional anti-human BCDF monoclonal antibodies.

The monoclonal antibody of the present invention may be prepared based on the so-called hybridoma method, well-known in the art, in which a hybridoma producing the monoclonal antibody of the present invention is cultured and the monoclonal antibody is recovered therefrom.

The hybridoma may be prepared by hybridizing a myeloma cell and an antibody-producing cell. The antibody-producing cell may be obtained by immunizing an animal such as mouse or rat with human BCDF and recovering antibody-producing cells, such as spleen cells or lymphocytes, from the immunized animal. The human BCDF to be administered to the animal may be recombinant human BCDF produced by a microorganism such as *E. coli,* or one produced by human tonsil monocyte or human peripheral blood monocyte or human tumor cells such as human T lymphoma, or may be one produced by an artificially prepared hybridoma. The method of recovering antibody-producing cells from the animal is well-known in the art.

The antibody-producing cells collected from the animal immunized with human BCDF are then hybridized with myeloma cells. Although the species of the animal from which the myeloma cell originated may be different from the species of the animal employed for the immunization, better results are usually obtained when the same species is used. An example of the preferred hybridoma for producing the monoclonal antibody of the present invention is a hybridoma between a mouse lymphoma or mouse spleen cell and a mouse myeloma cell. For example, a hybridoma between an antibody-producing lymphoma of BALB/c mouse immunized with human BCDF emulsified with Freund's complete adjuvant and mouse myeloma cell P3-X63-Ag8-U1 is preferred, as shown in the working examples described below. In place of the mouse myeloma cell P3-X63-Ag8-U1, 8-azaguanine resistant myeloma cells such as mouse P3-X63-Ag8 cell, mouse P3-NSI/1-Ag4-1 cell, mouse MPC11-45.6.TG.1.7 cell, mouse SP2/V-Ag14 cell, mouse X63-Ag8-6.5.3, rat 210.RCY.Ag1.2.3 cell, human SKO-007 cell and human GH15006TG-A12 cell, all of which are well-known in the art, may also be employed. The method of hybridization of the antibody-producing cell and the myeloma cell, which utilizes polyothyleneglycol or Sendai virus (HVJ), is well-known in the art.

After the cell hybridization, the cells are cultured in a HAT medium in which only hybridomas can survive so as to select the hybridomas. This culture is usually carried out in a 96-well microtiter plate. The hybridoma producing anti-human BCDF monoclonal antibody may be selected by checking the culture supernatant for the existence of the anti-human BCDF antibody. Checking is done by an immunoassay such as enzyme immunoassay utilizing the specific binding reaction between the antibody and human BCDF.

The anti-human BCDF monoclonal antibody of the present invention may be purified from the culture supernatant of the thus selected hybridoma by a conventional method such as salting out and ion-exchange chromotography. In cases where a large amount of the monoclonal antibody is to be produced, the hybridoma may be transplanted into the abdomen of a histocompatible animal such as nude mouse or the like, and the monoclonal antibody may be recovered from the abdominal fluid.

The present invention also provides an immunoassay for determining human BCDF level in a sample in which the monoclonal antibody of the present invention is specifically reacted with the human BCDF in the sample. The monoclonal antibody of the present invention may be employed in any immunoassay for quantifying human BCDF. As mentioned earlier, however, in a preferred mode of the present invention, the immunoassay uses a so-called sandwich method in which two anti-human monoclonal antibodies have corresponding epitopes which are different. Since an anti-human BCDF monoclonal antibody, which recognizes an epitope which is different from that recognized by the conventional anti-human BCDF monoclonal antibody, was first provided by the present invention, this sandwich immunoassay system employing two anti-human BCDF monoclonal antibody was also first provided by the present invention accordingly.

The sandwich immunoassay employing the monoclonal antibody of the present invention and a conventional anti-human BCDF monoclonal antibody may be carried out, for example, as follows. The anti-human BCDF monoclonal antibody of the present invention serving as a first antibody is fixed on the surface of a well. After blocking the surface of the well with a protein such as BSA, so as to prevent the non-specific binding, a sample which may contain human BCDF is placed in the well. After allowing it to react, the sample fluid is discarded and the well is washed. Then, labelled conventional anti-human monoclonal antibody such as MH166 or αBSF-2 serving as a second antibody is added to the well and the well is then washed. Any conventional labelling, such as labelling with radioisotope or enzyme, may be employed for labelling the second antibody. By quantifying the bound second antibody according to the label, the amount of the human BCDF may be determined. It should be noted that the conventional anti-human BCDF monoclonal antibody may be fixed on the well, and the monoclonal antibody of the present invention may be used as the secondary antibody.

The anti-human BCDF monoclonal antibody of the present invention may be used, in addition to the immunoassay, for immunohistochemical staining. This may be accomplished by employing an indirect enzyme antibody technique. That is, for example, after fixing a section of topical tissue under inflammation as a smear sample, the sample is blocked with a protein such as BSA so as to prevent the non-specific binding of the antibody, and anti-human BCDF monoclonal antibody of the present invention is then reacted with the sample. Then the sample is treated with a peroxidase-labelled anti-mouse Ig antibody and the resultant is left to stand to allow the reaction. After washing, the sample is then treated with 3,3-diaminobenzidine and hydrogen peroxide so as to color the sample. After treatment, the cells containing human BCDF therein are stained in brown.

The anti-human monoclonal antibody of the present invention may also be used as a reagent for recovering human BCDF. For example, the anti-human BCDF monoclonal antibody of the present invention is fixed on an appropriate support, such as resin, and affinity chromatography may be carried out using the antibody-bound resin. More particularly, the anti-human BCDF monoclonal antibody of the present invention may be fixed on a cellulose support activated with cyanogen bromide (commercially available from Pharmacia) and the support is then packed in a column so as to provide an affinity column. By applying a fluid containing human BCDF to the affinity column, and by subsequently eluting the human BCDF bound to the monoclonal antibody of the present invention fixed on the support, human BCDF with high purity may be obtained.

Further, as shown in the actual working examples hereinbelow described, since the monoclonal antibody of the present invention is capable of neutralizing the human BCDF activities, the monoclonal antibody of the present invention may be used for treating the autoimmune diseases, inflammation and some cancers in which the human BCDF participates.

It should be noted that F(ab')₂ fragment, prepared by removing Fc region from the anti-human BCDF monoclonal antibody of the present invention by an enzyme, may possibly also be used in the above-mentioned uses in place of the monoclonal antibody of the present invention. Further, a chimera antibody in which the Fc region of the anti-human BCDF monoclonal antibody of the present invention is exchanged with human Fc region may possibly also be used in place of the monoclonal antibody of the present invention.

The present invention will now be described by way of examples. It should be understood that the examples are presented for illustration purposes only and should not be interpreted in any restrictive way.

### Example 1

### 1) Preparation of Hybridoma Producing Anti-human BCDF Monoclonal Antibody

A BALB/c mouse (male, 8 weeks old) was immunized intraperitoneally with 2 - 3 µg of purified human BCDF produced by *E. coli,* which was admixed with equivolume Freund's complete adjuvant. The mouse was immunized another 4 - 6 times with the same antigen composition, and then its spleen was collected. A spleen cell suspension was prepared and the spleen cells were hybridized with P3-X63-Ag8-U1 (P3U1) by the conventional polyethyleneglycol method.

The hybridized cells were suspended in HAT medium and the suspension was separately placed in wells of a 96-well microtiter plate (commercially available from Corning Glass) with a population density of 2.5 x 10⁵ cells/well. After about two weeks from the hybridization, the culture supernatants in the wells in which growth of hybridomas was recognized were examined for the production of anti-human BCDF monoclonal antibody by the hybridomas by the following method: That is, recombinant human BCDF (2 µg/ml) produced by *E. coli* dissolved in PBS was separately placed in the wells of a 96-well microtiter plate, and was left to stand overnight at 4°C so as to fix the human BCDF in the wells. After removing unbound human BCDF, PBS containing 0.5% BSA was placed in the wells and was left to stand for 1 hour at room temperature. After removing the unbound BSA, culture supernatants of the hybridomas were separately placed in each well and were left to stand for 2 hours at room temperature. After washing the wells with PBS containing 0.05% Tween 20^{®}, anti-mouse Ig antibody labelled with peroxidase (commercially available from DAKO) was added to the wells and was left to stand for 2 hours at room temperature. After washing the wells with PBS containing 0.05% Tween 20^{®}, 2,2'-azino-bis(3-ethylbenzothiazoline-6-sulfuric acid)[ABTS] and hydrogen peroxide were added to the wells and the absorbance of the wells was measured. It was found that the hybridomas in two wells were producing anti-human BCDF monoclonal antibody, and each hybridoma was cloned to obtain two clones named HH61-8 and HH61-10. The specificities of the monoclonal antibodies HH61-8 and HH61-10 in terms of the absorbance of the wells are shown in Table 1 below.

**Table 1**

| Antibody | Absorbance |
|---|---|
| Normal Mouse IgG (control) | 0.016 |
| HH61-8 | 0.950 |
| HH61-10 | 1.278 |

### 2) Examination of whether or Not Anti-human BCDF Monoclonal Antibodies Recognize the Same Epitope

Whether or not the monoclonal antibody of the present invention recognizes the same epitope as the known anti-human BCDF monoclonal antibodies was examined by the following method: That is, BCDF diluted with PBS to a concentration of 10 µg/ml was placed in the wells of a 96-well microtiter plate (commercially available from Nunc) in the amount of 100 µl each and was left to stand overnight at 4°C. The solution in the wells was discarded and PBS containing 0.5% BSA was placed in each well in the amount of 200 µl/well, followed by being left to stand at room temperature for one hour. The solution in the wells was then discarded and 50 µl of about 1 µg/µl biotin-labelled anti-human BCDF monoclonal antibody solution in BSA and 50 µl of 100 µg/ml non-labelled human BCDF monoclonal antibody in BSA were placed in each well, followed by allowing to react at room temperature for 2 hours. After the reaction, the solution was discarded and each well was washed three times with PBS containing 0.5% Tween® 20 (PBS-Tween). Then 100 µl each of avidin solution and biotin-labelled alkaline phosphatase (Vectorstein ABC kit) were added to each well and the mixture was allowed to react for one hour at room temperature. Finally, each well was washed with PBS-Tween three times and 100 µl of 1 mg/ml p-nitrophenol phosphate solution was added to each well. The mixture was left to stand at room temperature until the mixture was appropriately colored, when the absorbance of the mixture at 405 nm was measured.

The results are shown in Table 2. In Table 2, the mark "+" indicates that binding inhibition occurred (i.e., the epitope of the monoclonal antibodies are identical) and the mark "-" indicates that binding inhibitation did not occur (i.e., the epitopes of the monoclonal antibodies are different). As can be seen from Table 2, the monoclonal antibodies HH61-8 and HH61-10 according to the present invention recognize an epitope which is different from that recognized by the conventional anti-human BCDF monoclonal antibodies MH166 and αBSF2-77.

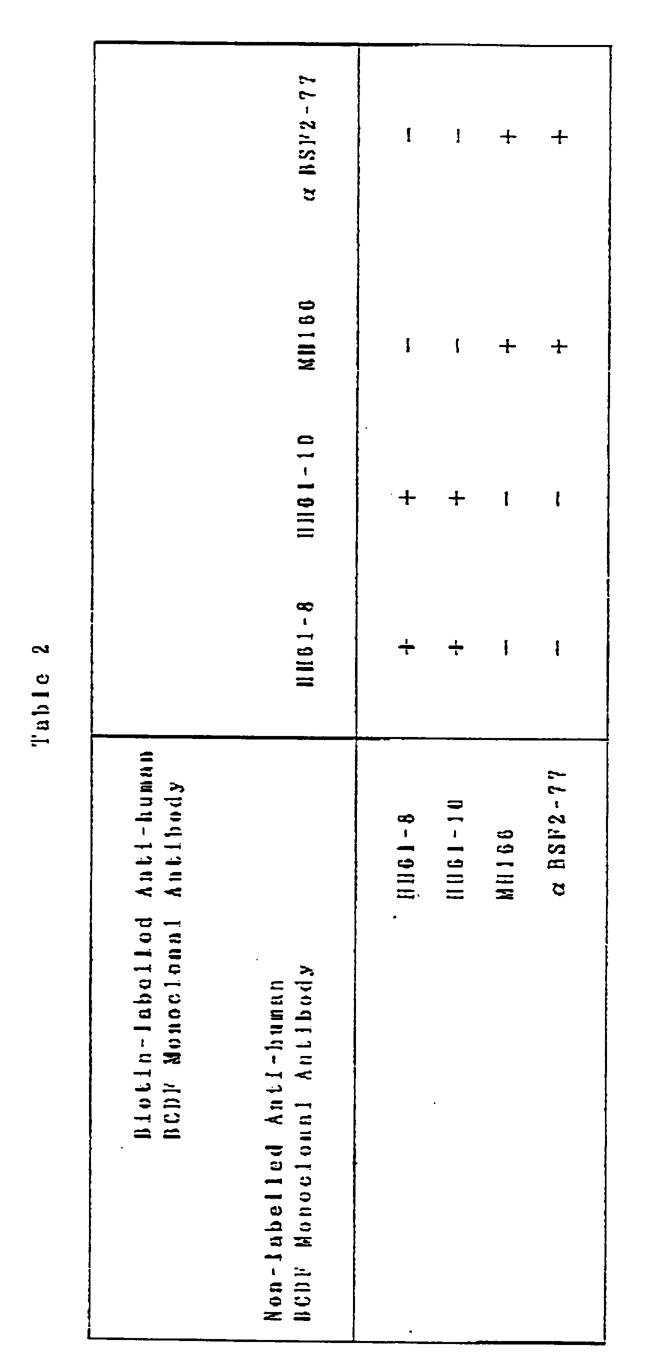

### Example 2

### Neutralization of Human BCDF Activity by Anti-human BCDF Monoclonal Antibodies

(1) To 5 µg/ml human BCDF solution placed in the wells of a 96-well microtiter plate, anti-human BCDF monoclonal antibodies HH61-8 or HH61-10 of the present invention were added in the amount of 100 - 0.05 µg/ml and the mixture was allowed to react for 1 hour at 37°C. To each reaction mixture, SKW6-CL-4 cells which are differentiated to IgM-producing cells in the presence of human BCDF were added in a population density of 1 x 10⁵ cells/ml and the resulting mixture was cultured for 72 hours. The culture supernatant in each well was then recovered and the IgM level was determined by the well-known enzyme immunoassay.

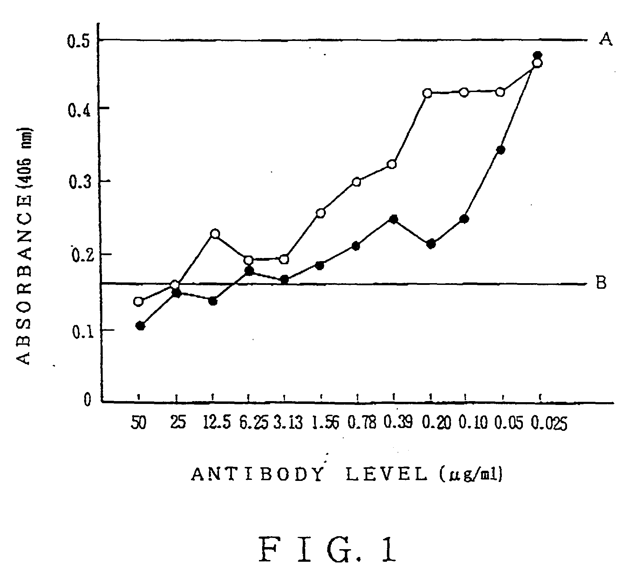

The results are shown in Fig. 1. In Fig. 1, the white circles indicate the results when the monoclonal antibody HH61-8 was used and the black circles indicate the results when the monoclonal antibody HH61-10 was used. Line A indicates the absorbance wherein no monoclonal antibody was added, and Line B indicates the background.

As can be seen from Fig. 1, monoclonal antibodies HH61-8 and HH61-10 specifically neutralized the human BCDF activity dose-dependently, and they completely neutralized the human BCDF activity at a concentration of 25 µg/ml or more.

### Example 3

### Preparation of Enzyme Immunoassay System Using Two Anti-human BCDF Monoclonal Antibodies Recognizing Different Epitopes

100 µl each of purified anti-human monoclonal antibody HH61-8 solution was placed in the wells of a 96-well microtiter plate, and left to stand overnight at 4°C. After removing the unbound anti-human BCDF monoclonal antibody, PBS containing 1.0% BSA was added to the each well and the mixture was left to stand overnight at 4°C. After removing the unbound BSA, 50 µl of standard human BCDF solution having a known concentration was added to each well and then 50 µl peroxidase-labelled anti-human BCDF monoclonal antibody MH166 was added to each well. The resulting mixture was left to stand for two hours at 37°C. After washing the wells, 100 µl of a solution of the substrate of peroxidase (ABTS-hydrogen peroxide) was added to each well and the mixture was left to stand for one hour at room temperature. The enzyme reaction was stopped by adding 100 µl each of 1% oxalic acid and the absorbance at 415 nm of each well was measured. The above-described measurement was performed using human BCDF solutions with various BCDF levels.

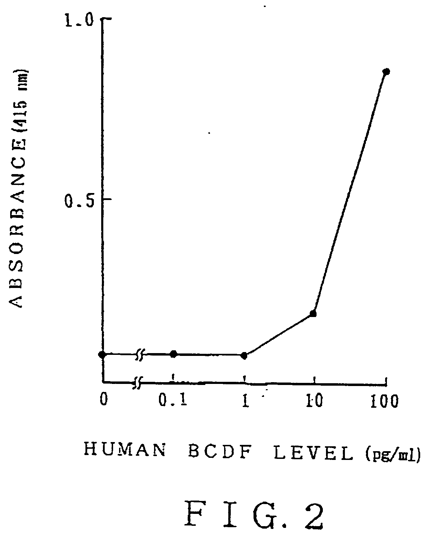

The results are shown in Fig. 2. As can be seen from Fig. 2, by the above-described enzyme immunoassay, human BCDF can be quantified at a level of about 5 pg/ml or higher.

## Claims

1. An anti-human BCDF monoclonal antibody having the epitope specificity of an antibody obtained from the hybridoma HH61-8 deposited as FERM BP-3029 or from the hybridoma HH61-10 deposited as FERM BP-3030.

2. An anti-human BCDF monoclonal antibody as obtainable from the hybridoma HH61-8 deposited as FERM BP-3029 or from the hybridoma HH61-10 deposited as FERM BP-3030.

3. The hybridomas HH61-8 and HH61-10 deposited as FERM BP-3029 and FERM BP-3030 respectively.

4. A process for producing the antibody of claim 1 or claim 2 which comprises culturing the hybridoma HH61-8 or HH61-10 deposited as FERM BP-3029 and FERM BP-3030 and recovering the antibody produced.

5. An immunoassay for determining human BCDF level in a sample comprising reacting human BCDF with a first anti-human BCDF monoclonal antibody which is said anti-human BCDF monoclonal antibody of claim 1 or claim 2 and determining the amount of human BCDF specifically bound to said anti-human BCDF monoclonal antibody.

6. The immunoassay of claim 4, wherein said determination of the amount of the specifically bound human BCDF is performed by a sandwich method which employs a second anti-human BCDF monoclonal antibody that recognizes an epitope which is different from that recognized by said anti-human BCDF monoclonal antibody.

7. The immunoassay of claim 5, wherein said first anti-human monoclonal antibody is said monoclonal antibody HH61-8 or HH61-10 and said second anti-human monoclonal antibody is said monoclonal antibody MH166 or αBSF2-77.

## Patentansprüche

1. Monoklonaler anti-human-BCDF-Antikörper mit der Epitopspezifität eines Antikörpers, der vom als FERM BP-3029 hinterlegten Hybridom HH61-8 oder vom als FERM BP-3030 hinterlegten Hybridom HH61-10 erhalten wird.

2. Monoklonaler anti-human-BCDF-Antikörper, wie vom als FERM BP-3029 hinterlegten Hybridom HH61-8 oder vom als FERM BP-3030 hinterlegten Hybridom HH61-10 erhältlich.

3. Als FERM BP-3029 bzw. FERM BP-3030 hinterlegte Hybridomen HH61-8 und HH61-10.

4. Verfahren zur Herstellung des Antikörpers nach Anspruch 1 oder 2, welches das Kultivieren des als FERM BP-3029 bzw. FERM BP-3030 hinterlegten Hybridoms HH61-8 oder HH61-10 und das Gewinnen des hergestellten Antikörpers umfaßt.

5. Immunoassay zum Bestimmen der human-BCDF-Menge in einer Probe, welches das Reagieren von human-BCDF mit einem ersten monoklonalen anti-human-BCDF-Antikörper, bei dem es sich um den monoklonalen anti-human-BCDF-Antikörper nach Anspruch 1 oder 2 handelt, und das Bestimmen der Menge an human-BCDF, das spezifisch an den monoklonalen anti-human-BCDF-Antikörper gebunden ist, umfaßt.

6. Immunoassay nach Anspruch 4, worin die Bestimmung der Menge des spezifisch gebundenen human-BCDF mit einem Sandwichverfahren erfolgt, bei dem ein zweiter monoklonaler anti-human-BCDF-Antikörper eingesetzt wird, der ein Epitop erkennt, das sich von dem unterscheidet, das vom monoklonalen anti-human-BCDF-Antikörper erkannt wird.

7. Immunoassay nach Anspruch 5, worin der erste monoklonale anti-human-Antikörper der monoklonale Antikörper HH61-8 oder HH61-10 ist und der zweite monoklonale anti-human-Antikörper der monoklonale Antikörper MH166 oder αBSF2-77 ist.

## Revendications

1. Anticorps monoclonal anti-BCDF humain ayant la spécificité d'épitope d'un anticorps obtenu de l'hybridome HH61-8 déposé en tant que FERM BP-3029 ou de l'hybridome HH61-10 déposé en tant que FERM BP-3030.

2. Anticorps monoclonal anti-BCDF humain tel qu'il peut être obtenu de l'hybridome HH61-8 déposé en tant que FERM BP-3029 ou de l'hybridome HH61-10 déposé en tant que FERM BP-3030.

3. Hybridomes HH61-8 et HH61-10 déposés en tant que FERM BP-3029 et FERM BP-3030, respectivement.

4. Procédé de production d'un anticorps de la revendication 1 ou de la revendication 2 qui comprend la mise en culture de l'hybridome HH61-8 ou HH61-10 déposé en tant que FERM BP-3029 et FERM BP-3030 et la récupération de l'anticorps produit.

5. Immunoanalyse pour déterminer le niveau de BCDF humain dans un échantillon consistant à faire réagir BCDF humain avec un premier anticorps monoclonal anti-BCDF humain qui est ledit anticorps monoclonal anti-BCDF humain de la revendication 1 ou de la revendication 2 et à déterminer la quantité de BCDF humain spécifiquement lié audit anticorps monoclonal anti BCDF humain.

6. Immunoanalyse de la revendication 4 où ladite détermination de la quantité de BCDF humain spécifiquement lié est accomplie par une méthode sandwich qui emploie un second anticorps monoclonal anti-BCDF humain qui reconnaît un épitope qui est différent de celui reconnu par ledit anticorps monoclonal anti-BCDF humain.

7. Immunoanalyse de la revendication 5 où ledit premier anticorps monoclonal anti-humain est ledit anticorps monoclonal HH61-8 ou HH61-10 et ledit second anticorps monoclonal anti-humain est ledit anticorps monoclonal MH166 ou αBSF2-77.
